# EUROPEAN PATENT SPECIFICATION

(11) **EP 1 696 900 B1**
(45) Date of publication and mention of the grant of the patent: **14.07.2010**
(21) Application number: 04814462.0
(22) Date of filing: 16.12.2004
(51) Int. Cl.: A61K 31/191, A61K 31/201, A61P 9/10

(54) **USE OF TREPROSTINIL TO TREAT ISCHEMIC LESIONS**
VERWENDUNG VON TREPROSTINIL ZUR BEHANDLUNG VON ISCHÄMISCHEN LÄSIONEN
UTILISATION DU TREPROSTINIL DANS LE TRAITEMENT DES LESIONS ISCHEMIQUES

(30) Priority: 16.12.2003 US 529622 P
(43) Date of publication of application: 06.09.2006
(73) Proprietor: United Therapeutics Corporation, Maryland 20910 (US)
(72) Inventor: WADE, Michael, c/o United Therapeutics Corp., Research Triangle Park, NC 27709 (US); JEFFS, Roger A., c/o United Therapeutics Corp., Research Triangle Park, NC 27709 (US); ROSCIGNO, Robert, c/o United Therapeutics Corp., Research Triangle Park, NC 27709 (US); STROOTMAN, Deborah, c/o United Therapeutics, Tucson, AZ 85743 (US); BRONSTEIN, Kathryn, Jupiter, FL 33478 (US)
(74) Representative: Ahner, Francis
(86) International application number: PCT/US2004/042283
(87) International publication number: WO 2005/058303

(56) References cited:
- WO-A-99/25357
- WO-A-2004/019952
- WO-A-2005/007081
- GB-A- 2 070 596
- US-A1- 2004 265 238
- CHUNG; FIORENTINO: '"Digital ulcers in patients with systemic sclerosis"' AUTOIMMUNITY REVIEWS 2006, pages 125 - 128
- CHUNG; FIORENTINO: 'A pilot trial of treprostinil?' AM. ACAD. OF DERMATOLOGY vol. 54, 2006, pages 880 - 882
- ENGEL; ROCKSON: 'Treprostinol for the treament of severe digital nezcrosis in systemic sclerosis' VASCULAR MEDICINE vol. 10, 2005, pages 29 - 32
- BERMAN; QUICK: 'Treprostinil sodium (remodulifor CLI)' VASCULAR vol. 14, no. 3, 2006, pages 142 - 148

## Description

### FIELD OF INVENTION

The invention relates to the use of Treprostinil or its derivative according to claim 1, or a pharmaceutically acceptable salt thereof, to treat and/or prevent ischemic lesions, such as digital (fingers and toes) ulcers and necrotic lesions, caused by scleroderma, Buerger's disease, Raynaud's disease, Raynaud's phenomenon or other conditions.

### BACKGROUND

Treprostinil, also known as UT-15, is a known compound disclosed in U.S. Pat. No. 4,306,075 in example 33. Treprostinil is a synthetic analog of epoprostenol, a prostaglandin F₁. The activities ascribed to the various compounds of this patent include inhibition of smooth muscle cell proliferation, inhibition of platelet aggregation, inhibition of cytokine secretion, reduction of gastric secretion, vasodialation and bronchodilation.

U.S. Pat. No. 5,153,222 discloses the use of Treprostinil and related compounds to treat pulmonary hypertension. U.S. Pat. No. 6,054,486 discloses the use of Treprostinil and related compounds to treat peripheral vascular disease, such as peripheral arterial occlusive disease and intermittent claudication. Patterson et al., Amer. J. of Cardiology, 75: 26A-33A (1995), have shown vasodilator effects of Treprostinil in patients with class III or class IV heart failure.

Clapp et al., Am. J. Respir. Cell. Mol. Biol., 26(2): 194-201 (2002), have shown that Treprostinil inhibits proliferation of human pulmonary arterial smooth muscle cells. Raychaudhuri et al,. J. Biol. Chem., 277(36): 33344-8 (2002), have disclosed that Treprostinil inhibits inflammatory cytokine (tumor necrosis factor-α, interleukin-1β, interleukin-6, and granulocyte macrophage colony-stimulating factor) secretion and gene expression by human alveolar macrophages.

Patients with diseases or conditions, such as scleroderma (including systemic sclerosis), experience, among other things, abnormalities in the blood vessels that supply the skin. As a result, these patients experience ulcerations or even areas of necrosis (tissue death) on certain parts of their skin. Ischemic lesions associated with diseases such as scleroderma tend to occur on the hands and fingers, often over the knuckles, but also on other bony prominences, such as elbows, knees, hips, ankles and toes.

To date, the standard of care for treatment of ischemic lesions has included administration of topical hydrocolloid dressings, topical antibiotic ointments, analgesics for pain, debridement and wound care for ischemic wounds. Although certain types of dressings sometimes can help to aid healing of the lesions, the these treatments are often unsuccessful.

Other investigators have suggested that Ilomedin, a stable prostacyclin analog, may heal ischemic ulcers in lower limbs, as seen in patients with Buerger's disease. Fiessinger and Schafer, Lancet, 335(8689): 555-7 (1990); Norgren et al., Eur. J. Vasc. Surg. (5): 463-7 (1990); Benthin, Ugeskr Laeger, 157(36): 4946-7 (1995). Others have suggested that patients treated with Ilomedin treatment may show improvements in the frequency and severity of Raynaud's attacks. Kyle et al., J Rheumatol., (9): 1403-6 (1992); McHugh et al., Ann Rheum Dis., 47(1): 43-7 (1988).

Mohler et al., Vascular Medicine, 5: 231-237 (2000) have demonstrated, in patients with severe intermittent claudication, that Treprostinil causes an increase in blood flow in large blood vessels of the lower limbs, such as the common femoral, superficial femoral, popliteal and anterial tibial arteries. These investigators also have found that Treprostinil stimulates detectable blood flow in ankles of certain peripheral arterial disease patients, who otherwise exhibited minimal or no detectable blood flow in the absence of treatment. Likewise, the investigators found that some patients show improved pulse volume recordings in lower limbs upon Treprostinil treatment.

Ischemic lesions, and particularly digital ischemic lesions, such as those caused by systemic schlerosis, are extremely painful, debilitating, and heal slowly. Thus, the need exists to identify viable methods, as well as kits, that can be used to prevent and treat such lesions. The present invention satisfies this need and provides related advantages as well.

### SUMMARY

Administration of Treprostinil or its derivatives according to the claims, or pharmaceutically acceptable salts thereof, reduces the occurrence, number, size and severity of ischemic lesions, including digital ischemic lesions (such as ulcers and necrotic lesions), present on subjects with diseases such scleroderma, Buerger's disease, Raynaud's disease, Raynaud's phenomenon, and other conditions. Treprostinil is well suited for the prevention and treatment of ischemic lesions, including digital ischemic lesions, because the compound is a stable analogue of prostaglandin, can be used in intravenous administration, is not degraded when it passes through the lungs, and has a long biological half-life.

Accordingly, present invention provides for the treatment or prevention of ischemic lesions, such as digital ischemic lesions, in subjects with scleroderma (including systemic schlerosis), Buerger's disease, Raynaud's disease, Raynaud's phenomenon, or other conditions, comprising administering to a subject in need thereof an effective amount of Treprostinil, its derivative according to the claims or a pharmaceutically acceptable salt thereof.

### BRIEF DESCRIPTION OF THE FIGURES

**FIG. 1** shows the design of a study that examines the use of Treprostinil for the treatment and prevention of digital ischemic lesions in patients with systemic sclerosis.
**FIG. 2** indicates the disposition of the patients enrolled in the study.
**FIG. 3** is a graph showing the size of target lesions during Treprostinil therapy.
**FIG. 4** is a graph showing the average improvement in diameter of baseline digital ischemic lesions. ,
**FIG. 5** is a bar graph showing the number of total and new digital ischemic lesions.
**FIG. 6** is a bar graph showing the subjective measures of digital ischemic lesions
**FIG. 7** shows the resolution of target digital ischemic lesions overlying 3^{rd} metacarpophalangeal (MCP).
**FIG. 8** shows patient assessed mean-average and worst rest pain rating.

### DETAILED DESCRIPTION

The inventors believe that therapies that enhance cutaneous blood flow (i.e., to the skin), by increasing blood flow though smaller vessels and capillaries, are effective to treat and prevent ischemic lesions on the skin, including digital ischemic lesions. Prostacyclins are small molecules that have been previously shown to cause dilation of large blood vessels, relaxation of smooth muscle, inhibition of smooth muscle proliferation, as well as inhibition of platelet aggregation, which is involved in the blood clotting process. Similar actions by Treprostinil at the microvascular level and on capillaries near the skin are believed to help enhance cutaneous blood flow and heal and/or prevent ischemia lesions or ulcers associated with scleroderma, Buerger's disease, Raynaud's disease, Raynaud's phenomenon, and other conditions.

The present invention relates to the treatment of ischemic lesions in a subject with a disease or condition that causes ischemic lesions, comprising administering to a subject in need thereof an effective amount of Treprostinil and/or a derivative thereof according to the claims and/or a pharmaceutically acceptable salt thereof. Suitable derivatives include acid derivatives according to claim 1, sustained release forms, inhaled forms and oral forms of Treprostinil,.

In one embodiment, the disease or condition that causes ischemic lesions comprises scleroderma, Buerger's disease, Raynaud's disease and/or Raynaud's phenomenon. In another embodiment, the ischemic lesions comprise digital ischemic lesions, such as finger ulcers and/or necrotic lesions. In another embodiment, the disease or condition that that causes ischemic lesions comprises systemic schlerosis. In an additional embodiment, pain and/or other symptoms associated with digital ischemic lesions are reduced, eliminated or prevented upon administration of an effective amount of Treprostinil and/or its derivatives according to claim 1, and/or pharmaceutically acceptable salts thereof.

Unless otherwise specified, the term "a" or "an" used herein shall mean "one or more."

The term "acid derivative" is used herein to describe C1-4 alkyl esters and amides, including amides wherein the nitrogen is optionally substituted by one or two C1-4 alkyl groups.

Further aspects of the present invention are concerned with the use of Treprostinil or its derivatives according to the claims, or pharmaceutically acceptable salts thereof, in the manufacture of a medicament for the treatment or prevention of ischemic lesions in subjects with Buerger's disease, scleroderma, Raynaud's disease, Raynaud's phenomenon, or other conditions.

In one embodiment, a Treprostinil sodium, is used currently marketed under the trade name of REMODULIN®. The FDA has approved Treprostinil sodium for the treatment pulmonary arterial hypertension by injection of dose concentrations of 1.0 mg/mL, 2.5 mg/mL, 5.0 mg/mL and 10.0 mg/mL. The chemical structure formula for Treprostinil sodium is: 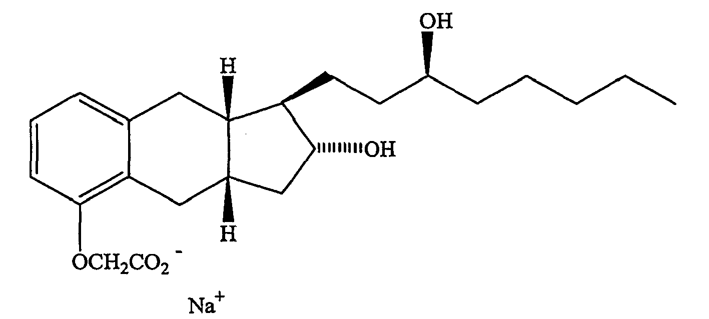 Treprostinil sodium is sometimes designated by the chemical names: (a) [(1*R*,2*R*,3a*S*,9a*S*)-2,3,3a,4,9,9a-hexahydro-2-hydroxy-1-[(3*S*)-3-hydroxyoctyl]-1*H-*benz[*f*]inden-5-yl]oxy]acetic acid; or (b) 9-deoxy-2',9-α-methano-3-oxa-4,5,6-trinor-3,7-(1',3'-interphenylene)-13,14-dihydro-prostaglandin F₁. Treprostinil sodium is also known as: UT-15; LRX-15; 15AU81; UNIPROST™; BW A15AU; and U-62,840. The molecular weight of Treprostinil sodium is 390.52, and its empirical formula is C₂₃H₃₄O₅.

The present invention extends to the use according to the claims of physiologically acceptable salts of Treprostinil, as well as non-physiologically acceptable salts of Treprostinil that may be used in the preparation of the pharmacologically active compounds of the invention.

Physiologically acceptable salts of Treprostinil include salts derived from bases. Base salts include ammonium salts (such as quaternary ammonium salts), alkali metal salts such as those of sodium and potassium, alkaline earth metal salts such as those of calcium and magnesium, salts with organic bases such as dicyclohexylamine and N-methyl-D-glucamine, and salts with amino acids such as arginine and lysine.

Quaternary ammonium salts can be formed, for example, by reaction with lower alkyl halides, such as methyl, ethyl, propyl, and butyl chlorides, bromides, and iodides, with dialkyl sulphates, with long chain halides, such as decyl, lauryl, myristyl, and stearyl chlorides, bromides, and iodides, and with aralkyl halides, such as benzyl and phenethyl bromides.

The amount of Treprostinil or its derivative according to the claims, or a pharmaceutically acceptable salt thereof, that is required in a medication or diagnostic aid according to the invention to achieve the desired effect will depend on a number of factors, such as the specific application, the nature of the particular compound used, the mode of administration, the concentration of the compound used, and the weight and condition of the patient. A daily dose per patient for treatment or prevention of ischemic lesions may be in the range 25 µg to 250 mg; 0.5 µg to 2.5 mg, or 7 µg to 285 µg, per day per kilogram bodyweight. For example, an intravenous dose in the range 0.5 µg to 1.5 mg per kilogram bodyweight per day may conveniently be administered as an infusion of from 0.5 ng to 1.0 µg per kilogram bodyweight per minute. One possible dosage is 2.5 ng/kg/min, increased over 12 weeks by an amount of 2.50 ng/kg/min each week, until a target dose, such as 15 ng/kg/min, is reached. Infusion fluids suitable for this purpose contain, for example, from 10 ng to 1 µg per milliliter. Ampoules for injection contain, for example, from 0.1 µg to 1.0 mg and orally administrable unit dose formulations, such as tablets or capsules, contain, for example, from 0.1 to 100 mg, typically from 1 to 50 mg. For diagnostic purposes, a single unit dose formulation may be administered. In the case of physiologically acceptable salts, the weights indicated above refer to the weight of the active compound ion, that is, the ion derived from Treprostinil.

In the manufacture of a medicament or diagnostic aid according to the invention, hereinafter referred to as a "formulation," Treprostinil and/or its derivatives, according to the claims and/or pharmaceutically acceptable salts thereof, may be admixed with, inter alia, an acceptable carrier. The carrier must, of course, be acceptable in the sense of being compatible with any other ingredients in the formulation and must not be deleterious to the subject. The carrier may be a solid or a liquid, or both, and is preferably formulated with the compound as a unit-dose formulation, for example, a tablet, which may contain from 0.05% to 95% by weight of the active compound. One or more of Treprostinil or its derivatives according to the claims, or pharmaceutically acceptable salts thereof, may be incorporated in the formulations of the invention, which may be prepared by any of the well known techniques of pharmacy for admixing the components.

In addition to Treprostinil, other pharmacologically active substances may be present in the formulations of the present invention which are known to be useful for treating ischemic lesions in subjects with scleroderma, Buerger's disease, Raynaud's disease, Raynaud's phenomenon, or other conditions. For example, the compounds of the invention may be present in combination with analgesics to treat pain, dressing changes, vasodilator medications, and topical or oral antibiotics.

The formulations of the invention include those suitable for parenteral (e.g., subcutaneous, intramuscular, intradermal, or intravenous), oral, inhalation (in solid and liquid forms), rectal, topical, buccal (e.g., sub-lingual) and transdermal administration, although the most suitable route in any given case may depend on the nature and severity of the condition being treated and on the nature of the particular form of Treprostinil, its derivative according to the claims, or a pharmaceutically acceptable salt thereof, which is being used.

Formulations of the present invention suitable for parenteral administration conveniently comprise sterile aqueous preparations of Treprostinil or its derivative according to the claims, or a pharmaceutically acceptable salt thereof, where the preparations may be isotonic with the blood of the intended recipient. These preparations may be administered by means of subcutaneous injection, although administration may also be effected intravenously or by means of intramuscular or intradermal injection. Such preparations may conveniently be prepared by admixing the compound with water or a glycine or citrate buffer and rendering the resulting solution sterile and isotonic with the blood. Injectable formulations according to the invention may contain from 0.1 to 5% w/v of active compound and may be administered at a rate of 0.1 ml/min/kg. Alternatively, the invention may administered at a rate of 0.625 to 50 ng/kg/min. Alternatively, the invention may be administered at a rate of 10 to 15 ng/kg/min.

Formulations suitable for oral administration may be presented in discrete units, such as capsules, cachets, lozenges, or tablets, each containing a predetermined amount of Treprostinil or its derivative, or a pharmaceutically acceptable salt thereof; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients).

In general, the formulations of the invention are prepared by uniformly and intimately admixing the active compound with a liquid or finely divided solid carrier, or both, and then, if necessary, shaping the resulting mixture. For example, a tablet may be prepared by compressing or molding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Molded tablets may be made by molding, in a suitable machine, the powdered compound moistened with an inert liquid binder.

Formulations suitable for buccal (sub-lingual) administration include lozenges comprising Treprostinil or its derivative, or a pharmaceutically acceptable salt thereof, in a flavored base, usually sucrose and acacia or tragacanth; and pastilles comprising the compound in an inert base such as gelatin and glycerin or sucrose and acacia.

Formulations suitable for rectal administration are preferably presented as unit dose suppositories. These may be prepared by admixing Treprostinil or its derivative, or a pharmaceutically acceptable salt thereof, with one or more conventional solid carriers, for example, cocoa butter, and then shaping the resulting mixture.

Formulations suitable for topical application to the skin preferably take the form of an ointment, cream, lotion, paste, gel, spray, aerosol, or oil. Carriers which may be used include vaseline, lanoline, polyethylene glycols, alcohols, and combinations of two or more thereof. The active compound is generally present at a concentration of from 0.1 to 15% w/w, for example, from 0.5 to 2% w/w. Formulations for transdermal administration may be delivered by iontophoresis (see, for example, *Pharmaceutical Research*, 3(6): 318 (1986)) and typically take the form of an optionally buffered aqueous solution of Treprostinil or its derivative or salt or thereof. Suitable formulations comprise citrate or bis/tris buffer (pH 6) or ethanol/water and contain from 0.1 to 0.2M active ingredient.

The compounds of the present invention are conveniently prepared by methods the same as or analogous to those described in U.S. Pat. No. 4,306,075, U.S. Pat. No. 6,528,688 and U.S. Pat. No. 6,441,245.

In certain embodiments, the disease or condition that causes ischemic lesions comprises scleroderma, Buerger's disease, Raynaud's disease and/or Raynaud's phenomenon. In one embodiment, the ischemic lesions comprise digital ischemic lesions. In another embodiment, pain or other symptom associated with digital ischemic lesions is reduced, eliminated or prevented. The digital ischemic lesions include finger ulcers and/or necrotic lesions. In one embodiment, the disease or condition that that causes ischemic lesions comprises systemic schlerosis.

In certain embodiments, the Treprostinil or its derivative according to the claims, or a pharmaceutically acceptable salt thereof, is administered subcutaneously, by continuous subcutaneous infusion, intravenously, in an orally available form selected from the group consisting of tablets and capsules, and/or by inhalation. In other embodiments, the effective amount of Treprostinil or its derivative according to the claims, or a pharmaceutically acceptable salt thereof, is at least 1.0 ng/kg of body weight/min.

In certain other embodiments, the disease or condition that causes ischemic lesions comprises systemic selerosis, and the ischemic lesions comprise digital ischemic lesions, and continuous administration of Treprostinil or its derivative according to the claims, or a pharmaceutically acceptable salt thereof, promotes the healing of at least one digital ischemic lesion, and reduces or prevents the development of new digital ischemic lesions. In another embodiment, a use for reducing, eliminating or preventing pain and disability associated with ischemic lesions (such as digital ischemic lesions) in a subject with a disease or condition that causes ischemic lesions comprises administering to a subject in need thereof an effective amount of Treprostinil or its derivative according to the claims, or a pharmaceutically acceptable salt thereof. In other embodiments, the subject is a human being, and the disease or condition that causes ischemic lesions comprises Buerger's disease that does not improve with smoking cessation. In another embodiment, the Treprostinil or its derivative according to the claims, or a pharmaceutically acceptable salt thereof, is administered by continuous subcutaneous infusion by an infusion pump.

### EXAMPLES

### Example 1

### Administration Of Treprostinil To Humans With Scleroderma Suffering From Digital Ischemic Lesions

Scleroderma patients having at least one lesion (i.e., small sore or area of tissue gangrene) present on a hand or finger are dosed with increasing amounts of Treprostinil over 12 weeks. The medication is delivered by a small pump that is connected to a catheter placed under the skin. In this manner, increasing dosages of Treprostinil are administered to patients by chronic continuous subcutaneous infusion.

Specifically, a 1.0 mg/mL formulation of Treprostinil sodium (REMODULIN^{®}) is administered subcutaneously using a standard micro-infusion, positive-pressure infusion pump designed for subcutaneous drug delivery (Mini-Med). Patients receive an initial dose of 2.5 ng/kg/min of study drug. If, in a given patient, a dose of 2.5 ng/kg/min is not tolerated (e.g., persistent headache, nausea, emesis, restlessness, anxiety or severe pain at infusion site that cannot be adequately managed by medication or topical treatment), the dose is reduced to 1.25 ng/kg/min. Patients are maintained at 2.5 ng/kg/min (or 1.25 ng/kg/min if 2.5 ng/kg/min is not tolerated) during Week 1. After that, the dose is raised by 2.50 ng/kg/min each week until not tolerated or once a target dose is reached.

Dosing is increased weekly unless not tolerated by the patient. Weekly dose increases do not exceed 2.50 ng/kg/min each. One example of a target dose is 15 ng/kg/min. The minimum dose is usually not less than 0.625 ng/kg/min. After completion of the Week 12 treatment, drug infusion are terminated by gradual reduction of the infusion rate (over a period of 1-4 hours, as clinically indicated) until a rate of 0 ng/kg/min is reached.

Patients receiving the above-described treatment experience fewer new lesions associated with scleroderma, and see a reduction in the number, size and severity of lesions present before treatment. The administration of Treprostinil treats and prevents digital ischemic lesions in patients with systemic sclerosis.

### Example 2

### Study of Treprostinil (Remodulin®) for the Treatment and Prevention of Digital Ischemic Lesions in Patients with Systemic Sclerosis

Digital ischemic lesions (DIL) occur in up to 35% of patients with systemic sclerosis and are exquisitely painful, often progressing to necrosis requiring amputation. The purpose of this study was to evaluate the effect of Treprostinil on the healing and prevention of DIL in patients with systemic sclerosis.

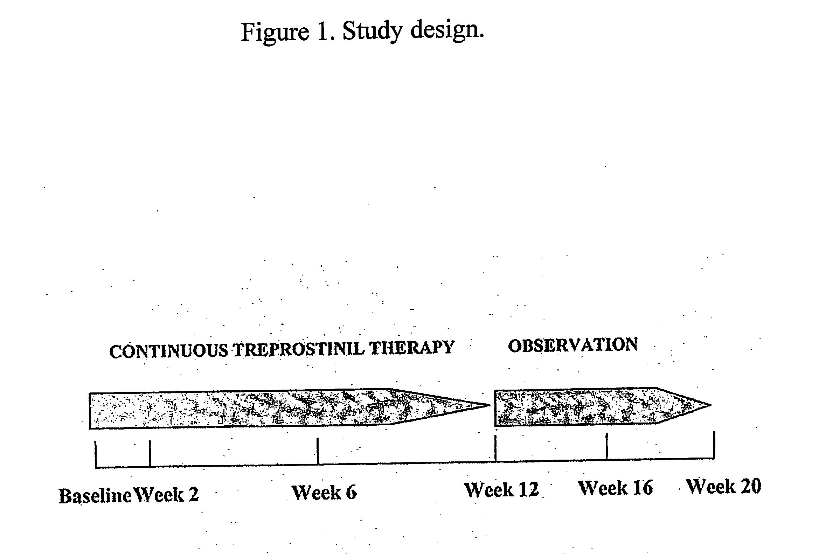

Methods: This study involved 12 subjects with diffuse or limited scleroderma with at least one DIL that had been present for 2 months or more (Table 1). Subjects who completed the study were treated for 12 weeks with Treprostinil and followed for another 8 weeks after drug discontinuation (FIG. 1).

**Table 1. Baseline Patient Demographics**

| Patient | 1 | 2 | 3 | 4 | 5 |
|---|---|---|---|---|---|
| Age (years) | 36 | 63 | 48 | 52 | 41 |
| Gender | Female | Female | Female | Female | Female |
| Limited v. Diffuse | Diffuse | Diffuse | Diffuse | Diffuse | Diffuse |
| Disease Duration (years) | 5.1 | 14.2 | 1.7 | 1.7 | 1.7 |
| Smoking History | Never | Never | Current | Remote¹ | Current |
| Antiphospholipid Antibodies | Yes | No | No | No | No |
| Other Risk Factors for Vasculopathy² | None | None | None | None | None |
| Concomitant Medications for Seleroderma (stable throughout study) | Nifedipine Losartan | Methotrexate Diltiazem Meloxicam Prednisone | Losartan Minocycline | Lisinopril Penicillaminc Minocycline Celecoxib | None |
| Number of DIL | 5 | 25 | 3 | 7 | 9 |
| Size of Target Lesion (mm) | 7 | 10 | 10 | 5 | 5 |

| | | | | | |
|---|---|---|---|---|---|
| ¹ Remote history of smoking if quit greater than 10 years ago. ² Risk factors assessed for at screening included a history of sickle cell disease, lymphoma, leukemia, myeloma, paraproteinemia, cryoglobulinemia, cryofibrinogenemia, hepatitis C infection, or diabetes mellitus. | | | | | |

Treprostinil (Remodulin®) was delivered to the subjects by continuous subcutaneous infusion, beginning at a rate of infusion of 2.5 ng/kg/min, which was increased by 2.5 ng/kg/min each week until a maximum rate of 15 ng/kg/min was achieved. Assessments were performed at baseline, weeks 2, 6, 12, 16, and 20. At each visit, the largest (target) lesion and other prominent DIL were measured by recording the largest diameter of the lesions. DIL were counted and photographed. Patient and physician global assessment of ulcers as well as patient assessment of disability from DIL were measured using visual analogue scales (VAS) at each visit.

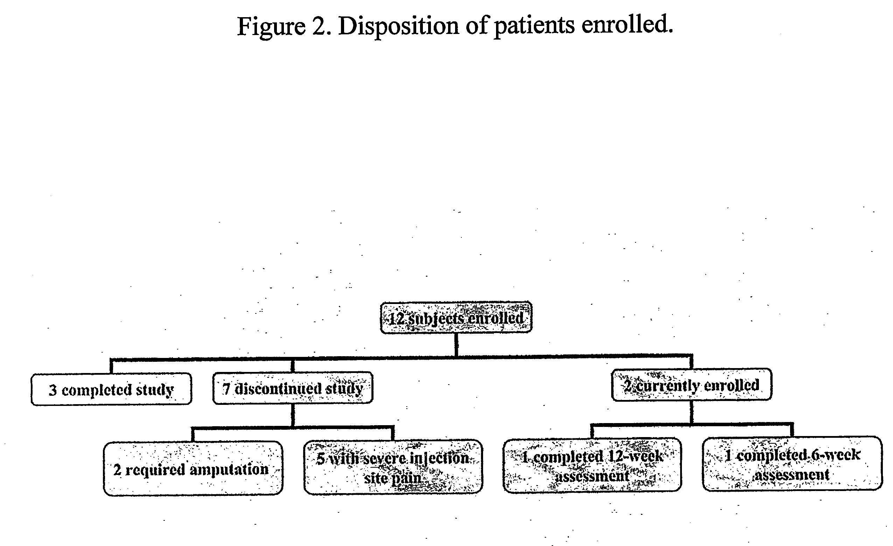

Results: Three of the 12 subjects completed the study and two are currently still enrolled (FIG. 2). Two subjects discontinued the study for surgical treatment of previously ischemic digits, and five subjects were unable to complete the study due to intolerable injection site pain (FIG. 2).

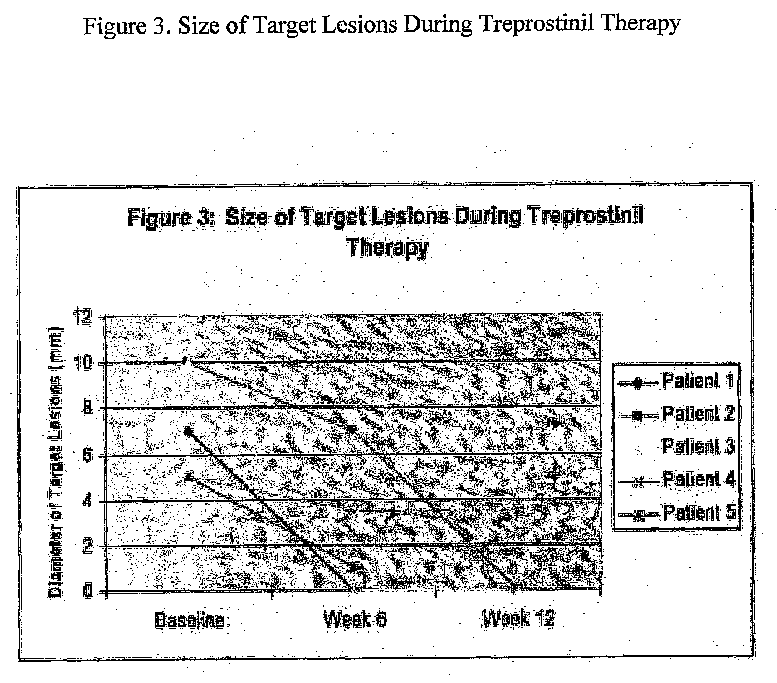

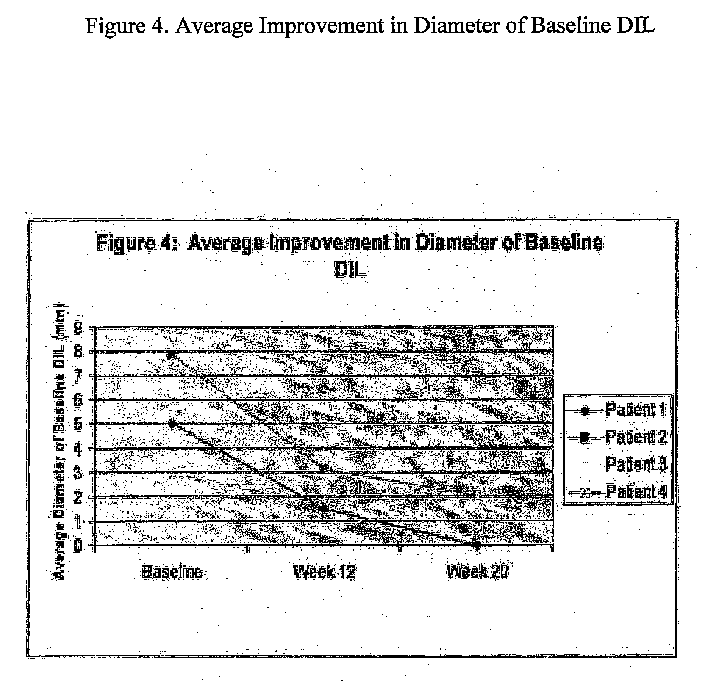

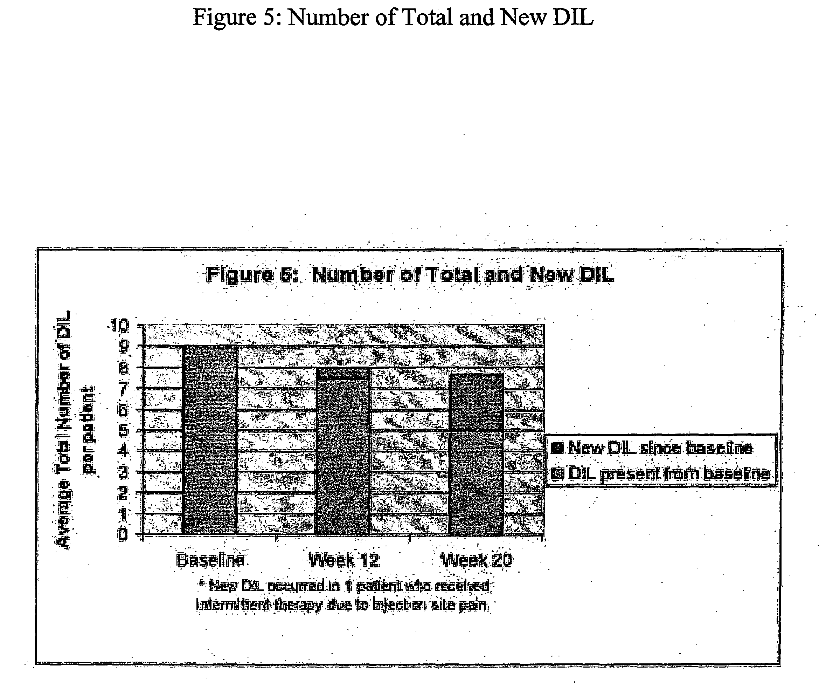

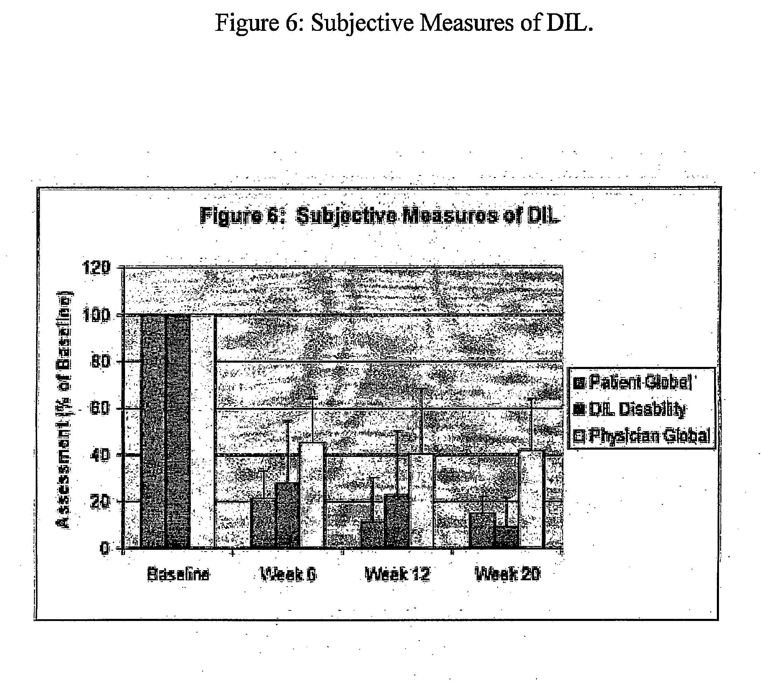

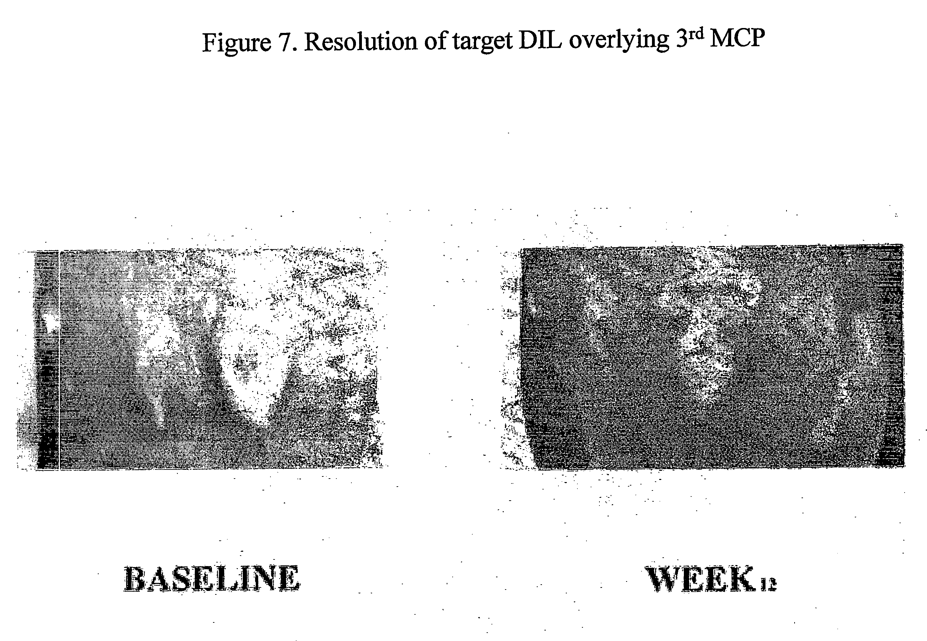

Of the four subjects who completed 12 weeks of active therapy, target lesions improved in all patients, and three experienced complete resolution of their target lesions (FIG. 3). On average there was a 65% decrease in the size of baseline DIL (FIG. 4). No new ulcers developed in any patients while receiving continuous Treprostinil therapy (FIG. 5); however, two of three patients developed new ulcers during the 8-week follow-up period after drug discontinuation. By week 6, all five subjects demonstrated marked improvements in subjective measures of severity of their DIL according to patient and physician global assessment and DIL disability VAS scores. Physician global assessment of DIL severity improved on average by 60% after 12 weeks of therapy (FIGS. 6 and 7). Patient global assessment and DIL disability VAS scores improved on average by 89% and 77% respectively by week 12 (FIGS. 6 and 7).

Conclusion: This study indicates that continuous subcutaneous Treprostinil therapy is useful in the treatment and prevention of DIL in patients with systemic sclerosis. Continuous Treprostinil therapy promotes healing of DIL, and is useful in preventing the development of new DIL. The Treprostinil therapy also reduces pain and disability associated with DIL.

### Example 3

### Treprostinil Sodium Provides Symptom Relief in Severe Buerger's Disease

### Background

Buerger's disease (*thromboangiitis oliterans* or TAO) is a clinical syndrome characterized by the development of segmental thrombotic occlusions of the medium and small arteries. The disease is clinically and pathologically distinguishable from atherosclerotic disease. Histopathology features may vary with the duration of the disease. In the chronic or end stage phase of the disease, only organized thrombus and fibrosis of the blood vessel is seen. In all stages of the disease, the normal structure of the vessel wall generally remains intact. Angiographic features of Buerger's disease are the involvement of small and medium sized vessels, segmental occlusive lesions, more severe disease distally and collateralization around areas of occlusion (corkscrew collaterals). Olin, Jeffery W., Current Concepts: Thromboangiitis Obliterans (Buerger's Disease), N. Engl. J. Med., Volume 343(12), 864-869 (September 21, 2000).

It is typically seen in young men who are heavy smokers and is more common in Asian and eastern European countries than in the US. Smoking is generally considered a requirement for diagnosis. Proposed clinical diagnostic criteria are: 1) smoking history, 2) onset before the age of 50 years; 3) infra-popliteal arterial occlusions; 4) either upper limb involvement or phlebitis migrans; and 5) absence of atherosclerotic risk factors other than smoking. Shionoya, Shigehiko Diagnostic criteria of Buerger's Disease, International Journal of Cardiology 66 (Suppl. 1) S243-S245 (1998).

The primary treatment for Buerger's disease is cessation of cigarette smoking. Persistent or recurrent symptoms occur rarely in patients who quit smoking and maintain a tobacco free environment to exclude any second-hand smoke. In patients whose disease progresses despite smoking cessation, therapeutic options are limited. Revascularization is rarely indicated and usually not successful because of the diffuse and distal distribution of the disease. Mills, Jopseph L Sr. Buerger's Disease in the 21st Century: Diagnosis, Clinical Features, and Therapy, Seminars in Vascular Surgery, Vol. 16(3), 179-189 (September 2003).

Treprostinil sodium (Remodulin®) is a stable analogue of prostacyclin with a plasma half life of more than 4 hours and is approved in the U.S. for chronic, continuous subcutaneous (SC) infusion in patients with pulmonary arterial hypertension (PAH). This case illustrates an example of a patients with severe and progressive Buerger's disease treated with a continuous subcutaneous infusion of treprostinil sodium in whom there were no other therapeutic options available.

### Case Report

A 42 year old Cuban male was first seen in 2002 for evaluation of ischemic pain of his right hand. The patient had a complicated medical history of bilateral foot gangrene resulting in a left BKA (below the knee amputation) in 1991 and a right BKA in 1993. His only risk factor was a long history of heavy cigarette smoking. He began to experience right hand pain in 2002. An arteriogram revealed right hand ischemia with few distal targets amenable for revascularization. A trial of thrombolytic therapy was attempted, but abandoned 48 hours later and the patient was discharged on warfarin. Because of recurrent ischemic ulcers and arm claudication, the patient sought additional opinions by several other vascular specialists and was told nothing could be done.

The patient's condition was diagnosed in 2002 as Buerger's disease. This patient met all the Buerger's diagnostic criteria with the exception of a positive history of hyperlipidemia which had not been present at the time he first developed symptoms. Review of systems was negative for connective tissue disease. On physical examination, both brachial pulses were palpable but bilateral radial and ulnar pulses were absent. There was evidence of chronic ischemic changes in the right hand with loss of the digital fat pads. Allens test was abnormal bilaterally. There was a small area of necrosis beneath the nail of the right thumb. There was another ischemic necrotic ulcer in the distal phalanx of the right middle finger just proximal to the nail which measured 1 cm in length. Both hands turned completely white and the patient would complain of pain with elevation of the arms.

The patient had a long history of smoking but quit in 2002 when his claudication and ischemic symptoms recurred. He has no history of diabetes or hypertension. He is a recovering alcoholic but denies illicit drug use. There is no family history of thrombotic disorders, or hypercoagulable disorders. Laboratory findings were negative for connective tissue diseases. A hypercoagulable lab panel, including factor V Leiden, antithrombin III, protein C, protein S, prothrombin gene mutation, anticardiolipin antibody, and lupus anticoagulant, was unremarkable.

Cilostazol was added to pentoxifylline, simvastatin and narcotic analgesics but symptoms did not improve. In December 2002, his right index finger was amputated due to gangrene. At follow-up, there was still significant necrosis and ulceration of the right thumb. The patient was referred to Anesthesia and underwent several stellate ganglion blocks, again with no reported change in symptoms. Eventually, the right thumb required amputation. He was lost to follow-up (i.e., was under another care provider) for a short period of time and an ulcer that developed on the right index finger became infected and subsequently amputated.

Soon after, the patient exhibited disabling claudication symptoms primarily manifest as weakness in both arms, especially the left, and unable to carry out simply activities of daily living such as dressing himself or combing his hair. The right middle finger ulcer was not healing.

Noninvasive vascular testing revealed flat tracings in both upper extremities at the digital level with the left worse than the right. An arteriogram showed occluded right brachial artery at the elbow with severe distal disease and an occluded left brachial artery at the takeoff from the axillary artery with severe disease of the left hand. The arteriogram demonstrated "corkscrew collaterals" at several levels. It was felt that the patient might benefit from revascularization and a left axillary brachial artery bypass using human umbilical vein was performed. Despite therapeutic anticoagulation, the bypass went on to occlude.

At this point, subcutaneous Treprostinil therapy was administered to the patient. Treprostinil was delivered chronically by continuous subcutaneous infusion using a pager-sized ambulatory infusion pump (Medtronic Minimed 407C, Minneapolis, MN)). In September 2003, Treprostinil was started at 2.5 ng/kg/min and titrated by 1 ng/kg/min every 7 days until the patient reached his maximum tolerated dose of 12.5 ng/kg/min and was continued for the next 10 months. He was unable to tolerate higher doses due to diarrhea and jaw pain, commonly reported dose limiting side effects of prostacyclin therapy. The patient has reported improved comfort and increased ability to participate in activities of daily living such as dressing self, combing his hair, reaching above his head and driving. Doppler studies demonstrated improvement in pulse volume recording wave form. Attempts to discontinue Treprostinil resulted in return of ischemic symptoms within 1 week. The patient is now on a maintenance dose of Treprostinil 12ng/kg/min from 9PM-9AM every seven day, with no drug for the next 7 days. The patient has had sustained relief of symptoms on this regimen including complete healing of the ulcer on his right middle finger.

The patient's symptomatic improvements appear to be related to Treprostinil infusion. The patient's disease continued to progress despite quitting smoking in early 2002. We confirmed the patient was smoke free with a negative cotinine urine test in 2003 at the time he was started on Treprostinil. There has been continued improvement in pain and digital ulcer healing and an overall improvement in his quality of life. While there are no formal dosing recommendations from the manufacturer, our dosing regimen including the maintenance dosing appears safe and effective based on clinical improvement.

These results suggest that subcutaneous Treprostinil therapy is clinically useful in Buerger's disease that does not improve with smoking cessation, particularly in the presence of critical limb ischemia where other therapeutic options have failed. The ease of the application, similar to insulin pumps, make it an attractive therapeutic option versus more invasive intravenous delivery and is well tolerated

### Example 4

### Treatment of Critical Limb Ischemia with Treprostinil Sodium (Remodulin®) Reduces Rest Pain and Heals Ischemic Ulcers.

Background: Treatment options are limited for patients with chronic critical limb ischemia (CLI), a life-and limb-threatening condition and the most severe form of peripheral arterial disease (PAD). Advanced CLI may lead to non-healing ischemic ulcer(s) and/or gangrene(Thrombosis Research 106(6): 295-301 (2002)).

The objectives of this study were an open-label, single-center evaluation of the safety and efficacy of continuous subcutaneous administration of treprostinil therapy in patients with CLI with no planned vascular interventional procedures and a determination of a safe dose of chronic treprostinil in these patients.

Methods: The planned enrollment was ten patients. All patients were to have Fontaine Stage III-IV or Rutherford Class 4-6 disease and ankle brachial indexes (ABI) from 0-0.55 in the most affected limb or the limb containing the reference ischemic wound for wound healing assessments. Patients were excluded from the study if they had a vascular surgery or vascular procedure within 30 days of study entry, were hemodynamically unstable, had acute renal failure, acute pulmonary failure, history of recent intracranial bleed, gastric bleeding urinary tract bleeding or significant trauma within 6 weeks, a life-threatening malignancy requiring aggressive chemotherapy, end-stage renal disease and chronic renal dialysis. Any condition or abnormal laboratory value which, based on information in the treprostinil package insert, would constitute an unacceptable risk to the patient's safety, also was an exclusion criterion. Patients could not have been in an ihvestigational trial within the past 30 days or been a non-responder to chronic prostanoid treatment in the past 30 days.

Medications for co-morbid, disorders such as coronary artery disease or COPD, normal wound care, including debridement and antibiotics, and analgesics for rest pain were permitted during the study but were not to be changed from the baseline regimens unless clinically necessary.

After the completion of baseline assessments, treprostinil therapy was initiated in the clinic. Patients were observed for at least two hours following the initiation of treprostinil therapy. Patients and/or a caregiver were trained to administer treprostinil on an outpatient basis using an ambulatory subcutaneous infusion pump (Minimed, Sylmar, CA, Model 407C). Each patient was to be initiated at a dose of 2.5 ng/kg/min or lower, with the dose titrated based on tolerability. Dose increases were to be 1.25-2.5 ng/kg/min per week. The maximum allowed dose was 15 ng/kg/min and the minimum allowed dose was 0.625 ng/kg/min. The patients were instructed to change the subcutaneous infusion site every three days.

Patients returned to the clinic for assessments at Weeks 2, 6, and 12. Treprostinil treatment was terminated by gradually decreasing the infusion rate (over a period of 1-4 hours, as clinically indicated) after the Week 12 visit assessments were completed.

Safety was assessed in all patients using adverse event (AEs) and physical examination findings. Signs and symptoms of CLI or worsening CLI were not considered to be AEs unless found to be different in causality, intensity, or frequency.

Rest pain was assessed in all patients using a visual analog scale (VAS) for rest pain. The patients were asked to rate their leg pain on a scale of 0-10 with 0 reflecting no pain and 10 reflecting the worst pain. The scale was printed and the patients were asked to place a mark on the number that reflected their pain experience. Patients were asked to rate the worst pain they had experienced since the previous assessment and their average pain during that time frame. Analgesic medication use was assessed by the investigator as unchanged, increased, decreased, or discontinued.

Wound assessments were to be conducted in patients who had at least one ischemic wound at baseline. If the patient had multiple ischemic wounds, then one or two (usually the largest or most severe wounds) were be selected as reference wounds. The selected wound(s) was photographed for documentation. When possible, the outside edge of the wound(s) was traced for area measurement. The tracings were used to calculate wound area by measuring the length and width of the wound. Not all wounds were of the nature that tracings were possible for example, wounds between toes or on the heel with extensive tissue loss were not traced. These wounds were described and photographed. The wound(s) was assessed for overall status compared to baseline (i.e., worse, slightly worse, unchanged,, slightly improved, improved or healed) at study visits.

In patients who had wounds other than those chosen as reference wounds, the overall status (i.e., worse, the same, improved, or healed) of each additional wound also was documented at each study visit. Any new wounds that occurred during the study also were carefully documented.

**Table 2. Patient Characteristics (n = 10)**

| Age | Range 65-90 | 82.4 (mean) |
|---|---|---|
| Sex | 4 males | 40% |
| CAD/CHF | 9 | 90% |
| Hypertension | 5 | 50% |
| TIA/Stroke | 3 | 30% |
| COPD | 2 | 20% |
| DM | 4 | 40% |
| Renal Insufficiency | 4 | 40% |
| GERD | 3 | 30% |
| | | |

| **Lesion sites** | | |
|---|---|---|
| SFA | 10 | 100% |
| Infra-popliteal | 7 | 70% |

### Results:

*Safety*: Ten patients (six females) were enrolled in the study after written consent. The mean age was 82.4 years and ranged from 65-90. Eight patients had established coronary artery disease, four were diabetic, and three had chronic renal insufficiency. All patients had diffuse PAD involving the superficial femoral artery (SFA). Infra-popliteal disease was present in 7 patients. Six patients had bilateral limb involvement. One patient had a previous below the knee amputation (BKA) due to PAD. Three patients had failed by-pass grafts and one had a failed angioplasty. All patients met criteria for Fontaine Stage IV (Rutherford 5 or 6) disease with ischemic rest pain and at least one ischemic limb wound. Table 3 summarizes the patient demographics and disease status.

All patients received subcutaneous treprostinil. All patients received an initial dose of 2.5 ng/kg/min of study drug. Nine patients were titrated to the maximum dose of 15 ng/kg/min between week 1-6. One patient elected to stay at 7.5 ng/kg/min due to severe site infusion pain.

The most common sided effect reported was infusion site pain. Two patients experienced mild jaw pain, one patient reported a mild headache and one patient experienced diarrhea. These side effects were resolved generally by decreasing the treprostinil dose. Two patients discontinued drug prematurely. One patient discontinued at week eight related to severe site pain, jaw pain, headache and diarrhea. One patient felt overwhelmed by the pump and infusion site changes and withdrew consent at week six but reported only mild infusion site pain.

There were two serious adverse event (SAEs). One female patient had a cholecystectomy at week 10 with normal post operative recovery. Treprostinil infusion was not discontinued during the laproscopic procedure. At week 12, this same patient developed worsening congestive heart failure requiring additional diuretics and the addition of an ACE inhibitor added to her medication regimen. Both SAEs were judged unlikely to be related to treprostinil.

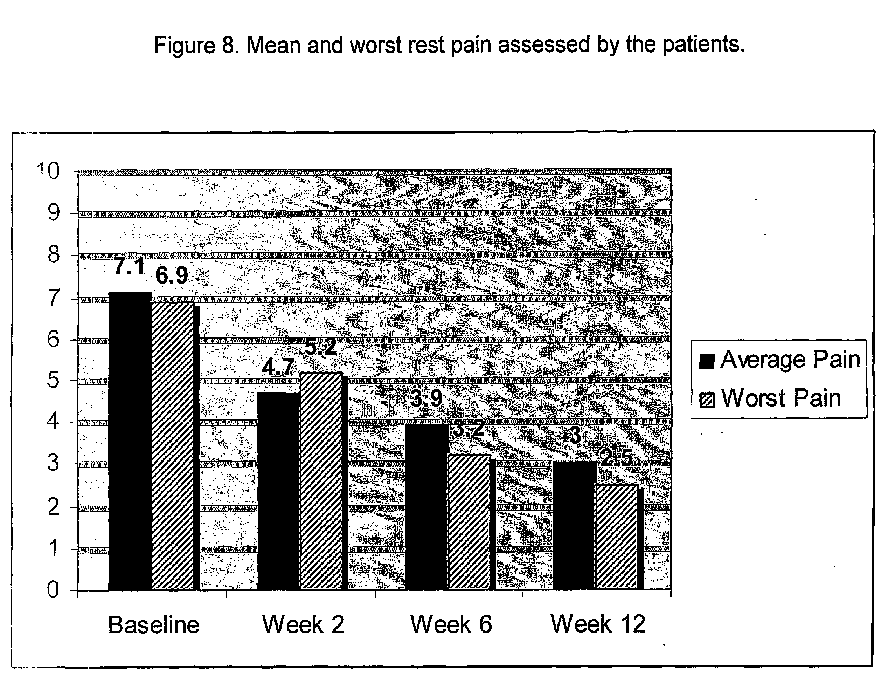

*Rest pain*: There was a 64% reduction in the worst rest pain from baseline to week 12 (from mean of 8.4 to 2.5) and a 58% reduction in average rest pain from baseline to week 12 (from a mean of 7.1 to 2.4). FIG. 8 shows patient-assessed mean average and worst rest pain rating on the visual analog scales at scheduled study visits and the mean average rest pain over time during the study.

**Table 3. Pain Medication Consumption**

| Patient | Basline Pain Medication(s) | Week 2 | Week 6 | Week 12 |
|---|---|---|---|---|
| 1 | Percocet | No change | No change | No change |
| 2 | Percocet | Less | Less | No change |
| 3 | Percocet | No change | Less | No change |
| 4 | Vicodin | Percocet | Discontinued | |
| 5 | Percocet | No change | Less | Darvocet |
| 6 | Vicodin | Less | Less | Percocet |
| 7 | Percocet | Less | Less | None |
| 8 | Percocet | No change | Increased | No change |
| 9 | Percocet | No change | No change | |
| 10 | Vicodin | Less | None | None |

At baseline, all patients were on either oxycodone HCL/acetaminophen (Percocet® Endo Labs Inc) or hydrocodone bitartrate/acetaminophen (Vicodin®, Abbott Laboratories Inc.) to manage ischemic rest pain. At week 12, one patient had increased her consumption of pain medication, 4 patients medication usage was unchanged from baseline, three patients had reduced their pain medication consumption, one patient switched to a non-sedating, non-narcotic pain medication and two patients experienced complete pain relief and discontinued all pain medications. The patient who discontinued the study because of infusion site pain had experienced complete ischemic pain relief and had discontinued pain medication at week 6, but resumed pain medication one week after discontinuing treprostinil.

**Table 4. Ischemic Wounds**

| **Patient** | **Reference Wound Location and description: Baseline** | **Wound Duration:** | **Wound Condition at 12 weeks** |
|---|---|---|---|
| **Patient** | **Reference Wound Location and description: Baseline** | **Wound Duration:** | **Wound Condition at 12 weeks** |
| 1 | Right Lateral Ankle No gangrene Exposed Tendon 5 cm² | 9 months | Slightly larger |
| 2 | Left lateral Lower leg No gangrene 44 cm² | 4 months | Slightly larger |
| 3 | L Heel large amount of tissue loss with necrosis 63.7 cm² | 3 months | Slightly larger |
| 4 | L dorsum of foot No Gangrene 15 cm² | 9 months | Partially healed |
| 5 | L 5^{th} Toe and documented osteomyelitis Able to probe to bone No gangrene 0.16 cm² | 2 months | Fully healed |
| 6 | Full thickness dry gangrene Left 3,4, and 5^{th} toes with large dorsal foot wound No measured | 3 months | No change |
| 7 | Ischemic breakdown R and L 3^{rd} toe No gangrene < 1.5 cm²* | 1 month | Fully Healed |
| 8 | Gangrenous ulceration tip of L 2 toe 1.87 cm² | 3 months | No Change |
| 9 | L ulcer medial aspect lower leg with cellulites No gangrene 3.5 cm² | 2 months | Partially healed at six weeks t |
| 10 | Neuropathic ulceration R Great Toe No gangrene 1.96 cm² | 1 year | Fully healed at 12 weeks |

*Wound healing:* Wound tracings and investigator rating (worse, unchanged, improved, or completely healed) were used to evaluate ischemic wounds. However, the nature and location of most wounds prevented wound tracing. Wounds varied in location, extent of tissue loss and degree of gangrene or necrosis. The investigator evaluation of worse, unchanged, improved or completely healed was used in the final evaluation. All ten patients had at least on ischemic wound at baseline. Wound duration varied from four weeks to nine months. Wound size ranged from 0.16 - 63.7 cm². Three patients experienced complete healing of their wounds. Patient 5 demonstrated complete wound closure at week 6 and patient 7 and 10 demonstrated complete wound closure at week 12. No patient developed a new wound during the trial. Brief case reports for these patients are presented below. A fourth case report is presented which represents a unique use for prostacyclin. Treprostinil was used to delay amputation to allow the patient to complete rehabilitation for a fractured hip on the endangered limb.

### Case 1

Patient 5 is an 88 year old female with peripheral vascular disease. An arteriogram shows a completely occluded left SFA with collaterals reconstituting the left popliteal artery. Her ABI at baseline was 0.30. She had a small ischemic ulcer on the left second toe for 2 months that measured 0.16 cm² and one could probe to the bone. An MRA noted osteomyelitis of the left second toe. She had complete wound closure at week 6. While her rest pain did not resolve completely, she changed from hydrocodone bitartrate/acetaminophen, to propoxyphene and acetaminophen. Her treprostinil dose was 15 ng/kg/min.

### Case 2

Patient 7 is an 88 year old female who presented with non-healing ischemic wounds on the right and left third toe following toenail removal 4 weeks previously. She had bilateral renal angioplasty with stints in 2003 . An arteriogram was deferred due to her renal status and creatinine of 2.7. The MRA showed diffuse infra-inguinal disease with two vessel run off to the foot. She was unable to walk any distance without leg pain and experienced severe ischemic rest pain. Her ABI at baseline were right 0.40 and left 0.36. At week 6 she had complete resolution of her rest pain, was able to walk without restrictions, and discontinued narcotic pain medication. At week 12 she had complete wound closure. Her treprostinil dose was 7.5 ng/kg/min.

### Case 3

Patient 10 is a 65 year old male, insulin dependent diabetic, chronic renal insufficiency, and congestive heart failure with 13 year history of PAD. He had a right femoral popliteal by-pass in 1991 and documented occlusion 5 months later. He has had repeated neuropathic ulcerations of the right great toe that have never fully resolved since 2001 in the presence of PAD. He participated in previous trial of another prostanoid in late 2001 and demonstrated improvement in ulcer at the completion of the trial but it is unknown if he was on placebo or active drug. He began experiencing ischemic rest pain in his right leg in 2003. At baseline, he had a non-healing ulcer on his right great toe ulcer for 9 months measuring 1.96 cm². He completed 12 weeks of treprostinil and showed early wound healing with complete wound closure at week 12. He also experienced complete resolution of his ischemic rest pain at week 2 as well as severe claudication symptoms and discontinued his narcotic pain medications. His treprostinil dose was 15 ng/kg/min.

### Case 4

Patient 3 is an 82 year old male with a history of oxygen dependent COPD, atrial fibrillation, hyperglycemia, anemia of unknown origin and multilevel vascular disease. His vascular disease history included transient ischemic attacks (TIA) requiring a carotid endarterectomy in 1995 and again in 2003, coronary artery disease requiring a coronary artery bypass in 1995, and documented peripheral artery disease since 2002. He broke his left hip in August 2003 and developed left heel and leg ischemic ulcers while in a rehabilitation facility. An ultrasound in November 2003 demonstrated distal right SFA stenosis, proximal left SFA mid SFA occlusion with large collaterals. Minimal flow was seen at the ankle level with toe pressure less than 40 mm/Hg. The right ABI was 0.58 and the left ABI was 0.25. The patient had two large ischemic wounds with extensive tissue loss located on the left heel (63.75 cm²) and left lateral leg (40.17 cm²). There was concern the patient would be unable to utilize a prosthetic limb following an amputation in the presence of the recent hip fracture and incomplete healing of the prosthetic hip. He was enrolled in the study to stabilize the wounds, provided rest ain relief, delay amputation and continue the rehabilitation of the left hip. His wounds remained stable during the twelve weeks of drug treatment with no significant improvement, however, no worsening. Average rest pain scores were 7 at baseline and reduced to 4. Worst rest pain scores reduced from 8 to 4. He reduced his pain med consumption from hydrocodone bitartrate/acetaminophen, and oxycontin to oxycodone HCL/acetaminophen alone. He was able to complete rehabilitation of his left hip and it is anticipated he will be able to utilize a prosthetic limb following a BKA as a result of this extra time for rehabilitation therapy.

Conclusions: This open-label study supports the safety of treprostinil infusion. The patients enrolled in this study reflected the demographics seen with this end stage presentation of PAD. This is a heterogeneous population with significant co-morbid disorders contributing to the overall disease process. These patients are the worst of the worst with impending amputations.

Ischemic pain and wounds are the primary management problem in patients with CLI. Treprostinil provided pain relief in all patients as well as wound healing in three patients. The patients who failed to demonstrate healing had large wounds with necrosis and/or gangrene. While the three patients who demonstrated complete healing had less tissue loss, one would anticipate they would have deteriorated given their extensive vascular disease and lack of surgical revascularization options.

It will be apparent to those skilled in the art that various modifications and variations can be made to the compositions and processes of this invention. Thus, it is intended that the present invention cover such modifications and variations, provided they come within the scope of the appended claims and their equivalents.

## Claims

1. Use of an effective amount of Treprostinil or its derivative, or a pharmaceutically acceptable salt thereof for the manufacture of a medicament for treating ischemic lesions in a subject with a disease or condition that causes ischemic lesions, wherein said derivative is a C1-4 alkyl ester or amide of Treprostinil, including amides wherein the nitrogen is optionally substituted by one or two C1-4 alkyl groups, a sustained release form of Treprostinil, an inhaled form of Treprostinil, an oral form of Treprostinil or a polymorph of Treprostinil.

2. The use of claim 1, wherein the disease or condition that causes ischemic lesions comprises scleroderma, Buerger's disease, Raynaud's disease and/or Raynaud's phenomenon.

3. The use of claims 1 or 2, wherein the ischemic lesions comprise digital ischemic lesions.

4. The use of claim 3, wherein the digital ischemic lesions comprise finger ulcers and/or necrotic lesions.

5. The use of claim 1, wherein the disease or condition that causes ischemic lesions comprises systemic schlerosis.

6. The use of claim 3, wherein pain or other symptom associated with digital ischemic lesions is reduced or eliminated.

7. The use of claim 1, wherein a pharmaceutically acceptable salt of Treprostinil or its derivative according to claim 1 is used.

8. The use of claims 1 to 7, wherein the subject is a human being.

9. The use of claims 1 to 8, wherein the medicament is intended for a subcutaneous administration.

10. The use of claims 1 to 8, wherein the medicament is intended for continuous subcutaneous infusion administration.

11. The use of claims 1 to 8, wherein the medicament is intended for intravenous administration.

12. The use of claims 1 to 8, wherein the medicament is in an orally available form selected from the group consisting of tablets and capsules.

13. The use of claims 1 to 8, wherein the medicament is intended for inhalation administration.

14. The use of claims 1 to 13, wherein the effective amount is at least 1.0 ng/kg of body weight/min.

15. The use of claim 1, wherein the disease or condition that causes ischemic lesions comprises systemic sclerosis, wherein ischemic lesions comprise digital ischemic lesions, and wherein the medicament is intended for continuous administration for promoting the healing of a least one digital ischemic lesion, and reducing the development of new digital ischemic lesions.

16. The use of an effective amount of Treprostinil or its derivative, or a pharmaceutically acceptable salt thereof for the manufacture of a medicament for reducing or eliminating pain and disability associated with ischemic lesions in a subject with a disease or condition that causes ischemic lesions, wherein said derivative is a C1-4 alkyl ester or amide of Treprostinil, including amides wherein the nitrogen is optionally substituted by one or two C1-4 alkyl groups, a sustained release form of Treprostinil, an inhaled form of Treprostinil, an oral form of Treprostinil or a polymorph of Treprostinil.

17. The use of claim 16, wherein the ischemic lesions comprise digital ischemic lesions.

18. The use of claim 2, wherein the subject is a human being, and wherein the disease or condition that causes ischemic lesions comprises Buerger's disease that does not improve with smoking cessation.

19. The use of claim 1, wherein the medicament is intended for continuous subcutaneous infusion administration by an infusion pump.

## Patentansprüche

1. Verwendung einer wirksamen Menge von Treprostinil oder seines Derivats oder eines pharmazeutisch unbedenklichen Salzes davon zur Herstellung eines Medikaments zur Behandlung von ischämischen Läsionen bei einem Probanden mit einer Krankheit oder Kondition, die ischämische Läsionen verursacht, wobei es sich bei dem Derivat um einen C1-4-Alkylester oder ein C1-4-Alkylamid von Treprostinil, umfassend Amide, wobei der Stickstoff optional durch eine oder zwei C1-4-Alkylgruppen substituiert ist, eine Form von Treprostinil mit retardierter Freisetzung, eine Inhalationsform von Treprostinil, eine orale Form von Treprostinil oder ein Polymorphes von Treprostinil handelt.

2. Verwendung nach Anspruch 1, wobei die Krankheit oder Kondition, die ischämische Läsionen verursacht, die Bürgersche Krankheit, die Raynaudsche Krankheit und/oder das Raynaudsche Phänomen umfasst.

3. Verwendung nach Anspruch 1 oder 2, wobei die ischämischen Läsionen digitale ischämische Läsionen umfassen.

4. Verwendung nach Anspruch 3, wobei die digitalen ischämischen Läsionen Fingergeschwüre und/oder nekrotische Läsionen umfassen.

5. Verwendung nach Anspruch 1, wobei die Krankheit oder Kondition, die ischämische Läsionen verursacht, systemische Sklerose umfasst.

6. Verwendung nach Anspruch 3, wobei Schmerzen oder andere Symptome in Verbindung mit digitalen ischämischen Läsionen verringert oder beseitigt werden.

7. Verwendung nach Anspruch 1, wobei ein pharmazeutisch unbedenkliches Salz von Treprostinil oder sein Derivat nach Anspruch 1 verwendet wird.

8. Verwendung nach Anspruch 1 bis 7, wobei der Proband ein Mensch ist.

9. Verwendung nach Anspruch 1 bis 8, wobei das Medikament für eine subkutane Verabreichung bestimmt ist.

10. Verwendung nach Anspruch 1 bis 8, wobei das Medikament zur Verabreichung durch subkutane Dauerinfusion bestimmt ist.

11. Verwendung nach Anspruch 1 bis 8, wobei das Medikament zur intravenösen Verabreichung bestimmt ist.

12. Verwendung nach Anspruch 1 bis 8, wobei das Medikament in einer oral verfügbaren Form ist, die aus der Gruppe bestehend aus Tabletten und Kapseln ausgewählt ist.

13. Verwendung nach Anspruch 1 bis 8, wobei das Medikament zur Verabreichung durch Inhalation bestimmt ist.

14. Verwendung nach Anspruch 1 bis 13, wobei die wirksame Menge wenigstens 1,0 ng/kg Körpergewicht/min beträgt.

15. Verwendung nach Anspruch 1, wobei die Krankheit oder Kondition, die ischämische Läsionen verursacht, systemische Sklerose umfasst, wobei die ischämischen Läsionen digitale ischämische Läsionen umfassen, und wobei das Medikament zur Dauerverabreichung zum Fördern der Heilung wenigstens einer digitalen ischämischen Läsion und Reduzieren der Entwicklung von neuen digitalen ischämischen Läsionen bestimmt ist.

16. Verwendung einer wirksamen Menge von Treprostinil oder seines Derivats oder eines pharmazeutisch unbedenklichen Salzes davon zur Herstellung eines Medikaments zum Verringern oder Beseitigen von Schmerzen und Gebrechen in Verbindung mit ischämischen Läsionen bei einem Probanden mit einer Krankheit oder Kondition, die ischämische Läsionen verursacht, wobei es sich bei dem Derivat um einen C1-4-Alkylester oder ein C1-4-Alkylamid von Treprostinil, umfassend Amide, wobei der Stickstoff optional durch eine oder zwei C1-4-Alkylgruppen substituiert ist, eine Form von Treprostinil mit retardierter Freisetzung, eine Inhalationsform von Treprostinil, eine orale Form von Treprostinil oder ein Polymorphes von Treprostinil handelt.

17. Verwendung nach Anspruch 16, wobei die ischämischen Läsionen digitale ischämische Läsionen umfassen.

18. Verwendung nach Anspruch 2, wobei der Proband ein Mensch ist, und wobei die Krankheit oder Kondition, die ischämische Läsionen verursacht, Bürgersche Krankheit umfasst, die sich bei einer Raucherentwöhnung nicht verbessert.

19. Verwendung nach Anspruch 1, wobei das Medikament zur Verabreichung durch subkutane Dauerinfusion durch eine Infusionspumpe bestimmt ist.

## Revendications

1. Utilisation d'une quantité effective de Treprostinil ou de son dérivé ou de son sel pharmacologiquement acceptable pour la fabrication d'un médicament prévu pour traiter des lésions ischémiques chez un sujet ayant une maladie ou un état qui provoque des lésions ischémiques, dans lequel ledit dérivé est un ester d'alkyle en C₁-C₄ ou un amide de Treprostinil, comprenant des amides, dans laquelle l'azote est facultativement remplacé par un ou deux groupes d'alkyle en C₁-C₄, une forme à libération prolongée du Treprostinil, une forme inhalée du Treprostinil, une forme orale du Treprostinil ou un polymorphe du Treprostinil.

2. Utilisation selon la revendication 1, dans laquelle la maladie ou l'état qui provoque des lésions ischémiques comprend la sclérodermie, la maladie de Buerger, la maladie de Raynaud et/ou le phénomène de Raynaud.

3. Utilisation selon les revendications 1 ou 2, dans laquelle les lésions ischémiques comprennent les lésions ischémiques digitales.

4. Utilisation selon la revendication 3, dans laquelle les lésions ischémiques digitales comprennent les ulcères des doigts et/ou les lésions nécrotiques.

5. Utilisation selon la revendication 1, dans laquelle la maladie ou l'état qui provoque des lésions ischémiques comprend la sclérose systémique.

6. Utilisation selon la revendication 3, dans laquelle la douleur ou un autre symptôme associé avec les lésions ischémiques digitales est réduit ou supprimé.

7. Utilisation selon la revendication 1, dans laquelle un sel pharmacologiquement acceptable de Treprostinil ou son dérivé selon la revendication 1 est utilisé.

8. Utilisation selon les revendications 1 à 7, dans lequel le sujet est un être humain.

9. Utilisation selon les revendications 1 à 8, dans laquelle le médicament est prévu pour une administration sous-cutanée.

10. Utilisation selon les revendications 1 à 8, dans laquelle le médicament est prévu pour une administration par perfusion sous-cutanée continue.

11. Utilisation selon les revendications 1 à 8, dans laquelle le médicament est prévu pour une administration intraveineuse.

12. Utilisation selon les revendications 1 à 8, dans laquelle le médicament se présente sous une forme orale choisie dans le groupe comprenant les comprimés et les gélules.

13. Utilisation selon les revendications 1 à 8, dans laquelle le médicament est prévu pour l'administration par inhalation.

14. Utilisation selon les revendications 1 à 13, dans laquelle la quantité effective est d'au moins 1,0 ng/kg de poids de corps/min.

15. Utilisation selon la revendication 1, dans laquelle la maladie ou l'état qui provoque les lésions ischémiques comprend la sclérose systémique, dans laquelle les lésions ischémiques comprennent les lésions ischémiques digitales et dans laquelle le médicament est prévu pour l'administration continue afin de favoriser la cicatrisation d'au moins une lésion ischémique digitale et réduire le développement de nouvelles lésions ischémiques digitales.

16. Utilisation d'une quantité effective de Treprostinil ou de son dérivé ou de son sel pharmacologiquement acceptable pour la fabrication d'un médicament prévu pour réduire ou supprimer la douleur et l'incapacité associées aux lésions ischémiques chez un sujet ayant une maladie ou un état qui provoque des lésions ischémiques, dans laquelle ledit dérivé est un ester d'alkyle en C₁-C₄ ou amide de Treprostinil comprenant les amides, dans laquelle l'azote est facultativement remplacé par un ou deux groupes d'alkyle en C₁-C₄, une forme à libération prolongée du Treprostinil, une forme inhalée du Treprostinil, une forme orale du Treprostinil ou un polymorphe du Treprostinil.

17. Utilisation selon la revendication 16, dans laquelle les lésions ischémiques comprennent les lésions ischémiques digitales.

18. Utilisation selon la revendication 2, dans laquelle le sujet est un être humain et dans laquelle la maladie ou l'état qui provoque les lésions ischémiques comprend la maladie de Buerger qui ne s'améliore pas avec l'arrêt du tabac.

19. Utilisation selon la revendication 1, dans laquelle le médicament est prévu pour l'administration par perfusion sous-cutanée continue avec une pompe à perfusion.
